(19) Europäisches Patentamt
European Patent Office
Office européen des brevets

(11) **EP 3 311 688 B1**

(12) **EUROPEAN PATENT SPECIFICATION**

(45) Date of publication and mention
of the grant of the patent:
**25.08.2021 Bulletin 2021/34**

(51) Int Cl.:
***A44B 18/00*** *(2006.01)* ***A61F 13/62*** *(2006.01)*

(21) Application number: **16811606.9**

(86) International application number:
**PCT/JP2016/067613**

(22) Date of filing: **14.06.2016**

(87) International publication number:
**WO 2016/204132 (22.12.2016 Gazette 2016/51)**

(54) **FEMALE MEMBER FOR TOUCH FASTENER**

AUFNAHMEELEMENT EINES KLETTVERSCHLUSSES

ÉLÉMENT FEMELLE POUR FERMETURE PAR CONTACT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.06.2015 JP 2015123455
09.06.2016 JP 2016115122**

(43) Date of publication of application:
**25.04.2018 Bulletin 2018/17**

(73) Proprietor: **Nitto Denko Corporation
Ibaraki-shi, Osaka 567-8680 (JP)**

(72) Inventors:
• **UCHIDA, Shou**
**Ibaraki-shi**
**Osaka 567-8680 (JP)**
• **IKISHIMA, Shinsuke**
**Ibaraki-shi**
**Osaka 567-8680 (JP)**
• **TAKEDA, Kohei**
**Ibaraki-shi**
**Osaka 567-8680 (JP)**
• **NAKAGAWA, Muneshige**
**Ibaraki-shi**
**Osaka 567-8680 (JP)**

(74) Representative: **Müller-Boré & Partner
Patentanwälte PartG mbB
Friedenheimer Brücke 21
80639 München (DE)**

(56) References cited:
**EP-A2- 2 191 801       WO-A1-97/19808
WO-A1-2007/097467   WO-A1-2008/130807
JP-A- H09 195 155     JP-A- 2001 000 212
JP-A- 2001 008 713    JP-A- 2002 315 607
JP-A- 2009 527 315    JP-A- 2012 183 316
US-A1- 2008 026 178**

**Description**

The present invention relates to a hook-and-loop fastener female member. The present invention also relates to a hook-and-loop fastener including the hook-and-loop fastener female member of the present invention. The present invention also relates to a sanitary article including the hook-and-loop fastener female member of the present invention.

Various hook-and-loop fasteners are proposed for materials for articles such as sanitary articles, for example, diapers and masks (see, for example, Patent Literatures 1 and 2). When used for, for example, a diaper, a hook-and-loop fastener female member is generally attached to a front surface of a waist portion of the diaper and allowed to engage with a hook-and-loop fastener male member (typically having an engaging hook) fixed to a side surface of the diaper, to thereby complete a hook-and-loop fastener. The hook-and-loop fastener is required to be able to be removed and attached many times.

In recent years, non-woven fabrics have been adopted for many of the engaging layers of hook-and-loop fastener female members (layers on which the engaging hooks of hook-and-loop fastener male members can engage) to be used for sanitary articles (in particular, disposable diapers, supporters, masks, and the like).

However, the related-art hook-and-loop fastener female member including the engaging layer adopting the non-woven fabric has a problem in that the hook-and-loop fastener female member does not have a sufficient engaging force with the hook-and-loop fastener male member.

WO 2008/130807 A1 describes a loop material for a loop and hook type fastener used in a disposable article or garment. US20008/0026178 A1 describes a loop-forming non-woven material for a mechanical closure element. WO 97/19808 A1 describes a creped hydroentangled non-woven laminate and a process for making the same. WO 2007/097467 A1 describes a non-woven web for a fastener female member.

[PTL 1] JP 2009-527315 A
[PTL 2] JP 2010-125337 A

The present invention has been made to solve the problems of the related art, and an object of the present invention is to provide a hook-and-loop fastener female member including an engaging layer adopting a non-woven fabric, the hook-and-loop fastener female member being excellent in engaging force with a hook-and-loop fastener male member. Another object of the present invention is to provide a hook-and-loop fastener including the hook-and-loop fastener female member of the present invention. Still another object of the present invention is to provide a sanitary article including the hook-and-loop fastener female member of the present invention.

A hook-and-loop fastener female member according to one embodiment of the present invention is a hook-and-loop fastener female member, comprising:

an engaging layer engageable with a hook-and-loop fastener male member; and
a physical property layer configured to hold the engaging layer,
in which the engaging layer includes a non-woven fabric of fiber,
wherein a material for the physical property layer is a non-woven fabric of fiber,
wherein the hook-and-loop fastener female member has an embossed pattern,
wherein the thickness of the welded portion formed by the embossed pattern is 100 $\mu$m or less, and
wherein the hook-and-loop fastener female member has a flexibility index A, which is expressed by Equation (1), of 0.004 g$^{-2}$ or more:

$$A=1/(P1 \times P2) \quad (1)$$

where:

P1 represents a maximum compressive load when a ring body, which is obtained by cutting the hook-and-loop fastener female member into a size of 25 mm in an MD direction by 50 mm in a CD direction, forming the cut hook-and-loop fastener female member into a ring shape with its physical property layer surface being a roll inner side and its CD being a circumference, and connecting a front end portion and a rear end portion of the hook-and-loop fastener female member to each other at two places through use of a stapler in such a manner that the end portions do not overlap each other so as to have a ring shape having a roll diameter of 20 mm, is deformed through application of a compressive load to the ring body from its axial direction at a speed of 50 mm/minute up to 14 mm; and
P2 represents a maximum compressive load when the ring body, which is obtained by cutting the hook-and-loop fastener female member into a size of 25 mm in an MD direction by 50 mm in a CD direction, forming the cut hook-and-loop fastener female member into a ring shape with its physical property layer surface being a roll inner side and its CD being a circumference, and connecting the front end portion and the rear end portion of the hook-and-loop fastener female member to each other at two places through use of a stapler in such a manner that the end portions do not overlap each other so as to have a ring shape having a roll diameter of 20 mm, is deformed through application of a compressive load to the ring body from its width direction by allowing a plastic plate to fall freely at a height of 35 mm down to 5 mm.

In one embodiment, the flexibility index A is from 0.01 $g^{-2}$ to 0.08 $g^{-2}$.

In one embodiment, the flexibility index A is from 0.02 $g^{-2}$ to 0.05 $g^{-2}$.

In one embodiment, the P1 is 70 g or less.

In one embodiment, the P1 is from 15 g to 40 g.

In one embodiment, the P2 is 4.05 g or less.

In one embodiment, the P2 is from 1 g to 2.5 g.

In one embodiment, a sum of a basis weight of the non-woven fabric in the engaging layer and a basis weight of a non-woven fabric in the physical property layer is 60 $g/m^2$ or less.

In one embodiment, the sum of the basis weight of the non-woven fabric in the engaging layer and the basis weight of the non-woven fabric in the physical property layer is from 30 $g/m^2$ to 47 $g/m^2$.

In one embodiment, the hook-and-loop fastener female member has a density of 110 $kg/m^3$ or less.

In one embodiment, the hook-and-loop fastener female member has a density of 50 $kg/m^3$ or less.

In one embodiment, the hook-and-loop fastener female member is formed only of non-woven fabrics.

In one embodiment, a ratio of an area of a welded portion formed by the embossed pattern to an area of an entire surface of the hook-and-loop fastener female member is 35% or less.

In one embodiment, the ratio of the area of the welded portion formed by the embossed pattern to the area of the entire surface of the hook-and-loop fastener female member is from 12% to 28%.

In one embodiment, the welded portion formed by the embossed pattern has a thickness of from 25 $\mu$m to 55 $\mu$m.

In one embodiment, the embossed pattern includes a discontinuous embossed pattern.

In one embodiment, a surface of the fiber forming the non-woven fabric included in the engaging layer and a surface of the physical property layer on an engaging layer side contain the same kind of polymer.

In one embodiment, the polymer includes polyolefin.

A hook-and-loop fastener according to one embodiment of the present invention includes: the hook-and-loop fastener female member according to the embodiment of the present invention; and a hook-and-loop fastener male member configured to engage with the hook-and-loop fastener female member.

A sanitary article according to one embodiment of the present invention includes the hook-and-loop fastener female member according to the embodiment of the present invention.

According to the present invention, the hook-and-loop fastener female member including an engaging layer adopting a non-woven fabric, the hook-and-loop fastener female member being excellent in engaging force with a hook-and-loop fastener male member, can be provided. The hook-and-loop fastener including such hook-and-loop fastener female member can also be provided. The sanitary article including such hook-and-loop fastener female member can also be provided.

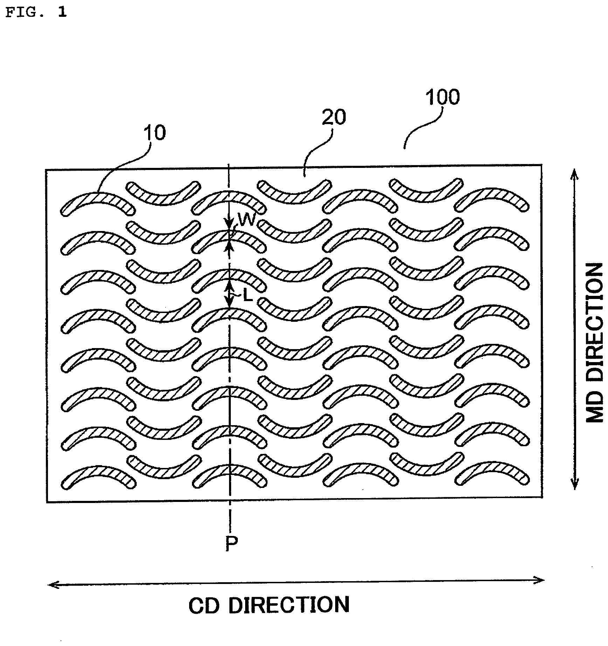

FIG. 1 is a schematic plan view of a hook-and-loop fastener female member according to a preferred embodiment of the present invention.

<<Hook-and-loop Fastener Female Member>>

A hook-and-loop fastener female member of the present invention is a hook-and-loop fastener female member including an engaging layer engageable with a male member (sometimes referred to as "mechanical hook member"). The engaging layer of the hook-and-loop fastener female member is specifically a layer on which an engaging hook (or something having properties equivalent to those of the engaging hook) of a hook-and-loop fastener male member is engageable. A product including the hook-and-loop fastener female member of the present invention and a hook-and-loop fastener male member configured to engage with the hook-and-loop fastener female member serves as a hook-and-loop fastener.

The hook-and-loop fastener female member of the present invention includes the engaging layer engageable with a hook-and-loop fastener male member and a physical property layer configured to hold the engaging layer. The hook-and-loop fastener female member of the present invention may include any appropriate other member as long as the hook-and-loop fastener female member includes such engaging layer and physical property layer, and the effect of the present invention is not impaired. The hook-and-loop fastener female member of the present invention is preferably formed of the engaging layer engageable with a hook-and-loop fastener male member and the physical property layer conf igured to hold the engaging layer.

The thickness of the hook-and-loop fastener female member of the present invention may be set to any appropriate thickness depending on the purpose. Typically, the thickness of the hook-and-loop fastener female member of the present invention is preferably from 0.2 mm to 5.0 mm, more preferably from 0.3 mm to 4.0 mm, still more preferably from 0.5 mm to 3.0 mm, particularly preferably from 0.5 mm to 2.0 mm. In the present invention, the thickness of the hook-and-loop fastener female member (non-woven fabric) is measured on the basis of a method to be described later.

The engaging layer includes a non-woven fabric of fiber. The number of layers of the engaging layer may be only one, or may be two or more. The engaging layer is preferably formed only of the non-woven fabric of fiber.

The number of kinds of the non-woven fabric of fiber included in the engaging layer may be only one, or may be two or more.

Examples of the non-woven fabric of fiber included in the engaging layer include a spunbonded non-woven fabric, a thermally bonded non-woven fabric, a bonded and joined non-woven fabric, an air-through non-woven fabric, a meltblown non-woven fabric, a spunbonded meltblown spunbonded non-woven fabric, a spunbonded meltblown meltblown spunbonded non-woven fabric, an unjoined non-woven fabric, an electrospun non-woven fabric, a flashspun non-woven fabric (e.g., TYVEK™ from DuPont), and a carded non-woven fabric. Of the above-mentioned non-woven fabrics, a spunbonded non-woven fabric, a thermally bonded non-woven fabric, a bonded and joined non-woven fabric, an air-through non-woven fabric, a meltblown non-woven fabric, a spunbonded meltblown spunbonded non-woven fabric, or a spunbonded meltblown meltblown spunbonded non-woven fabric is preferred, a spunbonded non-woven fabric or an air-through non-woven fabric is more preferred, and a spunbonded non-woven fabric is still more preferred. When, for example, a thermal point-bonded spunbonded non-woven fabric or an air-through non-woven fabric is used as the non-woven fabric of fiber included in the engaging layer, pieces of the fiber forming the non-woven fabric included in the engaging layer can have mutual bonding points. With this, when the hook-and-loop fastener female member of the present invention has an embossed pattern, not only pieces of the fiber forming the non-woven fabric included in the engaging layer have firm mutual bonding points in the embossed pattern portions as a result of embossing treatment, but also pieces of the fiber forming the non-woven fabric included in the engaging layer have mutual bonding points in a region free of the embossed pattern. When such structure can be achieved, the hook-and-loop fastener female member of the present invention is a hook-and-loop fastener female member including an engaging layer adopting a non-woven fabric, the hook-and-loop fastener female member being excellent in engaging force with a hook-and-loop fastener male member.

In the case where the non-woven fabric of fiber included in the engaging layer is a spunbonded non-woven fabric, the number of bonding points per unit area to be recognized in the region free of the embossed pattern in observation of the non-woven fabric of fiber included in the engaging layer with an optical microscope in a 17 mm×13 mm field of view (at a magnification of 7.5) is preferably from 10 to 200, more preferably from 30 to 150, still more preferably from 50 to 100. In the case where the non-woven fabric of fiber included in the engaging layer is a spunbonded non-woven fabric, when the number of bonding points per unit area to be recognized in observation of the non-woven fabric of fiber included in the engaging layer with an optical microscope falls within the above-mentioned range, fluffing of the engaging layer after peeling after engagement with a hook-and-loop fastener male member can be more effectively suppressed in the hook-and-loop fastener female member of the present invention.

In the case where the non-woven fabric of fiber included in the engaging layer is an air-through non-woven fabric, the number of bonding points per unit area to be recognized in the region free of the embossed pattern in observation of the non-woven fabric of fiber included in the engaging layer with an SEM in a 1.3 mm×1.0 mm field of view (at a magnification of 100) is preferably 1 or more, more preferably from 2 to 100, still more preferably from 5 to 50. In the case where the non-woven fabric of fiber included in the engaging layer is an air-through non-woven fabric, when the number of bonding points per unit area to be recognized in observation of the non-woven fabric of fiber included in the engaging layer with an SEM falls within the above-mentioned range, fluffing of the engaging layer after peeling after engagement with a hook-and-loop fastener male member can be more effectively suppressed in the hook-and-loop fastener female member of the present invention.

The non-woven fabric of fiber included in the engaging layer may contain fiber that is a homogeneous structural body, or may contain composite fiber that is a bicomponent structural body, such as a core-sheath structure, a side-by-side structure, a sea-island structure, or any other bicomponent structure. Detailed descriptions of the non-woven fabric may be found in, for example, "Nonwoven Fabric Primer and Reference Sampler," E.A. Vaughn, Association of the Nonwoven Fabrics Industry, third edition (1992) .

Any appropriate fiber may be adopted as the fiber forming the non-woven fabric included in the engaging layer as long as the effect of the present invention is not impaired. For example, such fiber contains polyolefin (such as polypropylene or polyethylene), polyester, polyamide, polyurethane, an elastomer, rayon, cellulose, acrylic, a copolymer thereof, or a blend thereof, or a mixture thereof . Such fiber includes preferably at least one kind selected from fiber of polyolefin (polyolefin fiber), fiber of polyester (polyester fiber), and composite fiber of two or more kinds of resins selected from polyolefin and polyester because the effect of the present invention can be more effectively expressed.

Examples of the polyolefin fiber include polypropylene fiber, polyethylene fiber, and α-olefin copolymer fiber. The polyolefin fiber is preferably polypropylene fiber or polyethylene fiber, more preferably polypropylene fiber because the effect of the present invention can be more effectively expressed.

Examples of the polyester fiber include polyethylene terephthalate (PET) fiber, polylactic acid fiber, and polyglycolic acid fiber. The polyester fiber is preferably polyethylene terephthalate (PET) fiber because the effect of the present invention can be more effectively expressed.

Examples of the composite fiber of two or more kinds of resins selected from polyolefin and polyester include fiber having a core-sheath structure, fiber having a side-by-side structure, and hollow fiber. As used herein, the term "composite fiber of two or more kinds of resins selected from polyolefin and polyester" means composite fiber of resins that are two or more kinds of polyolefin, composite fiber of resins that are two or more kinds of polyester, or composite fiber of resins that are one or more kinds of polyolefin and one or more kinds of polyester.

Specific examples of the composite fiber of two or more kinds of resins selected from polyolefin and polyester include: fiber having a core-sheath structure in which its core portion contains one of two kinds of polyolefin and its sheath portion contains the other; fiber having a core-sheath structure in which its core portion contains polyester and its sheath portion contains polyolefin; and fiber in which polyolefin and polyester form a side-by-side structure.

The fiber forming the non-woven fabric included in the engaging layer may be crimpable fiber. An example of the crimpable fiber is fiber containing two components having different freezing points, the fiber having a side-by-side structure or an unevenly distributed core-sheath structure, the fiber expressing fine coiled crimps each having a relatively small radius because the component having the higher freezing point first solidifies and shrinks at the time of a phase change from a molten state to a solid state.

The fiber forming the non-woven fabric included in the engaging layer may contain any appropriate other component as long as the effect of the present invention is not impaired. Examples of such other component include other polymers, a tackifier, a plasticizer, an antidegradant, a pigment, a dye, an antioxidant, an antistatic agent, a lubricant, a blowing agent, a heat stabilizer, a light stabilizer, an inorganic filler, and an organic filler. Those components may be used alone or in combination thereof. The content of the other component in the fiber forming the non-woven fabric included in the engaging layer is preferably 10 wt% or less, more preferably 7 wt% or less, still more preferably 5 wt% or less, particularly preferably 2 wt% or less, most preferably 1 wt% or less.

In the hook-and-loop fastener female member of the present invention, the density of the non-woven fabric in the engaging layer is preferably from 5 $kg/m^3$ to 100 $kg/m^3$, more preferably from 10 $kg/m^3$ to 100 $kg/m^3$, still more preferably from 10 $kg/m^3$ to 80 $kg/m^3$, still more preferably from 10 $kg/m^3$ to 70 $kg/m^3$, particularly preferably from 10 $kg/m^3$ to 60 $kg/m^3$, most preferably from 20 $kg/m^3$ to 50 $kg/m^3$, because the effect of the present invention can be more effectively expressed. In the hook-and-loop fastener female member of the present invention, when the density of the non-woven fabric in the engaging layer falls within the above-mentioned range, the engaging force with a hook-and-loop fastener male member is more excellent, and hence, in a disposable diaper or the like, the problem of slippage, for example, at the time of wearing or after excretion can be effectively eliminated. In the hook-and-loop fastener female member of the present invention, when the density of the non-woven fabric in the engaging layer is lower than 5 $kg/m^3$, there is a fear that a hook-and-loop fastener male member may be hardly hooked or productivity may be poor, leading to an increased cost. When the density of the non-woven fabric in the engaging layer is higher than 100 $kg/m^3$, a state in which the fiber of the non-woven fabric of the hook-and-loop fastener female member is densely packed is established, and hence there is a fear that it may be difficult to insert the engaging portion of a hook-and-loop fastener male member into the hook-and-loop fastener female member and an excellent engaging force cannot be expressed. In the present invention, the density ($kg/m^3$) of the non-woven fabric in the engaging layer is a value calculated from the basis weight (X $g/m^2$) of the non-woven fabric and the thickness (Y mm) of the non-woven fabric measured on the basis of a method to be described later. More specifically, the density ($kg/m^3$) of the non-woven fabric in the engaging layer is calculated as X/Y ($kg/m^3$).

In the hook-and-loop fastener female member of the present invention, the diameter of the fiber (hereinafter sometimes referred to simply as "fiber diameter") of the non-woven fabric in the engaging layer is preferably from 5 μm to 60 μm, more preferably from 10 μm to 60 μm, still more preferably from 10 μm to 50 μm, still more preferably from 10 μm to 40 μm, particularly preferably from 15 μm to 40 μm, most preferably from 20 μm to 40 μm, because the effect of the present invention can be more effectively expressed. In the hook-and-loop fastener female member of the present invention, when the diameter of the fiber of the non-woven fabric in the engaging layer falls within the above-mentioned range, the engaging force with a hook-and-loop fastener male member is more excellent, and hence, in a disposable diaper or the like, the problem of slippage, for example, at the time of wearing or after excretion can be effectively eliminated. In the hook-and-loop fastener female member of the present invention, when the diameter of the fiber of the non-woven fabric in the engaging layer is smaller than 5 μm, there is a fear that the engaging force with a hook-and-loop fastener male member may lower. When the diameter of the fiber of the non-woven fabric in the engaging layer is larger than 60 μm, there is a fear that engagement with a hook-and-loop fastener male member may become difficult or production speed may lower, leading to an increased cost. In the present invention, the diameter of the fiber of the non-woven fabric in the engaging layer (fiber diameter) is measured on the basis of a method to be described later.

In the hook-and-loop fastener female member of the present invention, the basis weight of the non-woven fabric in the engaging layer is preferably from 10 g/m$^2$ to 60 g/m$^2$, more preferably from 12 g/m$^2$ to 50 g/m$^2$, still more preferably from 15 g/m$^2$ to 40 g/m$^2$, particularly preferably from 15 g/m$^2$ to 30 g/m$^2$, most preferably from 15 g/m$^2$ to 25 g/m$^2$. In the hook-and-loop fastener female member of the present invention, when the basis weight of the non-woven fabric in the engaging layer falls within the above-mentioned range, there can be provided a hook-and-loop fastener female member including an engaging layer adopting a non-woven fabric, the hook-and-loop fastener female member being more excellent in engaging force with a hook-and-loop fastener male member.

Generally, any appropriate material may be adopted as a material for the physical property layer as long as the effect of the present invention is not impaired. General examples of the material for the physical property layer include a non-woven fabric of fiber and a film. According to the present invention, the material for the physical property layer is a non-woven fabric of fiber because the effect of the present invention can be more effectively expressed. That is, the hook-and-loop fastener female member of the present invention is preferably formed only of non-woven fabrics because the effect of the present invention can be more effectively expressed.

The material for the physical property layer is a non-woven fabric of fiber, and the number of kinds of the non-woven fabric may be only one, or may be two or more.

The material for the physical property layer is a non-woven fabric of fiber, and examples of the non-woven fabric include a spunbonded non-woven fabric, a thermally bonded non-woven fabric, a bonded and joined non-woven fabric , an air-through non-woven fabric, a meltblown non-woven fabric, a spunlace non-woven fabric, a spunbonded meltblown spunbonded non-woven fabric, a spunbonded meltblown meltblown spunbonded non-woven fabric, an unjoined non-woven fabric, an electrospun non-woven fabric, a flashspun non-woven fabric (such as TYVEKTM from DuPont), and a carded non-woven fabric.

The material for the physical property layer is a non-woven fabric of fiber, and the non-woven fabric may contain fiber that is a homogeneous structural body, or may contain composite fiber that is a bicomponent structural body, such as a core-sheath structure, a side-by-side structure, a sea-island structure, or any other bicomponent structure. Detailed descriptions of the non-woven fabric may be found in, for example, "Nonwoven Fabric Primer and Reference Sampler, " E.A. Vaughn, Association of the Nonwoven Fabrics Industry, third edition (1992).

The material for the physical property layer is a non-woven fabric of fiber, and any appropriate fiber may be adopted as the fiber as long as the effect of the present invention is not impaired. For example, such fiber contains polyolefin (such as polypropylene or polyethylene), polyester, polyamide, polyurethane, an elastomer, rayon, cellulose, acrylic, a copolymer thereof, or a blend thereof, or a mixture thereof. Such fiber includes preferably at least one kind selected from fiber of polyolefin (polyolefin fiber), fiber of polyester (polyester fiber), and composite fiber of two or more kinds of resins selected from polyolefin and polyester because the effect of the present invention can be more effectively expressed.

Examples of the polyolefin fiber include polypropylene fiber, polyethylene fiber, and $\alpha$-olefin copolymer fiber. The polyolefin fiber is preferably polypropylene fiber or polyethylene fiber, more preferably polypropylene fiber because the effect of the present invention can be more effectively expressed.

Examples of the polyester fiber include polyethylene terephthalate (PET) fiber, polylactic acid fiber, and polyglycolic acid fiber. The polyester fiber is preferably polyethylene terephthalate (PET) fiber because the effect of the present invention can be more effectively expressed.

Examples of the composite fiber of two or more kinds of resins selected from polyolefin and polyester include fiber having a core-sheath structure, fiber having a side-by-side structure, and hollow fiber. As used herein, the term "composite fiber of two or more kinds of resins selected from polyolefin and polyester" means composite fiber of resins that are two or more kinds of polyolefin, composite fiber of resins that are two or more kinds of polyester, or composite fiber of resins that are one or more kinds of polyolefin and one or more kinds of polyester.

Specific examples of the composite fiber of two or more kinds of resins selected from polyolefin and polyester include: fiber having a core-sheath structure in which its core portion contains one of two kinds of polyolefin and its sheath portion contains the other; fiber having a core-sheath structure in which its core portion contains polyester and its sheath portion contains polyolefin; and fiber in which polyolefin and polyester form a side-by-side structure.

The material for the physical property layer is a non-woven fabric of fiber, and the fiber forming the non-woven fabric may be crimpable fiber. An example of the crimpable fiber is fiber containing two components having different freezing points, the fiber having a side-by-side structure or an unevenly distributed core-sheath structure, the fiber expressing fine coiled crimps each having a relatively small radius because the component having the higher freezing point first solidifies and shrinks at the time of a phase change from a molten state to a solid state.

The material for the physical property layer is a non-woven fabric of fiber, and the fiber forming the non-woven fabric may contain any appropriate other component as long as the effect of the present invention is not impaired. Examples of such other component include other polymers, a tackifier, a plasticizer, an antidegradant, a pigment, a dye, an antioxidant, an antistatic agent, a lubricant, a blowing agent, a heat stabilizer, a light stabilizer, an inorganic filler, and an organic filler. Those components may be used alone or in combination thereof. The content of the other component in the fiber forming the non-woven fabric included in the engaging layer is preferably 10 wt% or less, more preferably 7 wt% or less, still more preferably 5 wt% or less, particularly preferably 2 wt% or less, most preferably 1 wt% or less.

In a general embodiment outside the scope of the present invention, when the material for the physical property layer is a film, any appropriate material may be adopted as a material for the film as long as the effect of the present invention is not impaired. Examples of such material include an unstretched polypropylene film, a stretched polypropylene film, and a polyethylene film each having a thickness of from 10 $\mu$m to 60 $\mu$m.

The physical property layer is a non-woven fabric of fiber, and the non-woven fabric in the physical property layer has a basis weight of preferably from 10 g/m$^2$ to 40 g/m$^2$, more preferably from 10 g/m$^2$ to 30 g/m$^2$, still more preferably from 10 g/m$^2$ to 25 g/m$^2$, particularly preferably from 10 g/m$^2$ to 20 g/m$^2$. When the basis weight of the non-woven fabric in the physical property layer falls within the above-mentioned range, shrinkage deformation in a width direction hardly occurs during web handling, cost competitiveness is excellent, printability is satisfactory, the see-throughproperty of printing is satisfactory, pressure-sensitive adhesive application is easy, the applied pressure-sensitive adhesive hardly exudes to the engaging surface, and flexibility is satisfactory.

The physical property layer is a non-woven fabric of fiber, and the diameter of the fiber is preferably 40 $\mu$m or less, more preferably from 1 $\mu$m to 40 $\mu$m, still more preferably from 1 $\mu$m to 30 $\mu$m, particularly preferably from 1 $\mu$m to 25 $\mu$m, most preferably from 1 $\mu$m to 20 $\mu$m. When the diameter of the fiber falls within the above-mentioned range, shrinkage deformation in a width direction hardly occurs during web handling, cost competitiveness is excellent, printability is satisfactory, the see-through property of printing is satisfactory, pressure-sensitive adhesive application is easy, the applied pressure-sensitive adhesive hardly exudes to the engaging surface, and flexibility is satisfactory. When the diameter of the fiber is larger than 40 $\mu$m, there is a fear that shrinkage deformation in a width direction may be liable to occur during web handling, cost competitiveness may be inferior, printability may be poor, pressure-sensitive adhesive application may be difficult, and the applied pressure-sensitive adhesive may have a risk of exuding to the engaging surface.

In the hook-and-loop fastener female member of the present invention, the density of the non-woven fabric is preferably from 5 kg/m$^3$ to 200 kg/m$^3$, more preferably from 20 kg/m$^3$ to 150 kg/m$^3$, still more preferably from 50 kg/m$^3$ to 150 kg/m$^3$, still more preferably from 50 kg/m$^3$ to 120 kg/m$^3$, particularly preferably from 60 kg/m$^3$ to 120 kg/m$^3$, most preferably from 70 kg/m$^3$ to 120 kg/m$^3$, because the effect of the present invention can be more effectively expressed. In the present invention, the density (kg/m$^3$) of the non-woven fabric in the physical property layer is a value calculated from the basis weight (X g/m$^2$) of the non-woven fabric and the thickness (Y mm) of the non-woven fabric measured on the basis of a method to be described later. More specifically, the density (kg/m$^3$) of the non-woven fabric in the physical property layer is calculated as X/Y (kg/m$^3$).

The physical property layer is a non-woven fabric of fiber, and a laminate of different kinds of non-woven fabrics of fiber (e.g., a laminate of spunbonded non-woven fabric/meltblown non-woven fabric/spunbonded non-woven fabric) may be adopted.

When the diameter of the fiber significantly varied in a thickness direction (e.g., SMS or SSMMS), the same number of diameters of the fiber were measured for N=5 or more in each piece of the fiber, and the average value of the measured diameters was defined as the diameter of the fiber. A portion having a locally small thickness due to heat fusion or the like as in spunbond, spunmelt, or the like was not included.

According to a general embodiment outside the scope of the present invention, when the physical property layer is a film, its thickness is preferably 60 $\mu$m or less, more preferably from 10 $\mu$m to 50 $\mu$m, still more preferably from 10 $\mu$m to 40 $\mu$m, particularly preferably from 10 $\mu$m to 30 $\mu$m, most preferably from 15 $\mu$m to 25 $\mu$m. When its thickness falls within the above-mentioned range, shrinkage deformation in a width direction hardly occurs during web handling, cost competitiveness is excellent, the see-through property of printing is satisfactory, pressure-sensitive adhesive application is easy, and flexibility is satisfactory. When its thickness is larger than 60 $\mu$m, there is a fear that cost competitiveness may be inferior, the see-through property of printing may degrade, and flexibility may degrade.

In the hook-and-loop fastener female member of the present invention, the total basis weight, which is the sum of the basis weight of the non-woven fabric in the engaging layer and the basis weight of the non-woven fabric in the physical property layer, is preferably 60 g/m$^2$ or less, more preferably from 10 g/m$^2$ to 57 g/m$^2$, still more preferably from 15 g/m$^2$ to 53 g/m$^2$, particularly preferably from 20 g/m$^2$ to 50 g/m$^2$, most preferably from 30 g/m$^2$ to 47 g/m$^2$, In the hook-and-loop fastener female member of the present invention, when the total basis weight, which is the sum of the basis weight of the non-woven fabric in the engaging layer and the basis weight of the non-woven fabric in the physical property layer, falls within the above-mentioned range, the engaging force with a hook-and-loop fastener male member is more excellent. In addition, in the hook-and-loop fastener female member of the present invention, when the total basis weight, which is the sum of the basis weight of the non-woven fabric in the engaging layer and the basis weight of the non-woven fabric in the physical property layer, falls within the above-mentioned range, shrinkage deformation in a width direction is still less liable to occur during web handling, cost competitiveness is more excellent, printability is more satisfactory, the see-through property of printing is more satisfactory, pressure-sensitive adhesive application is easier, the applied pressure-sensitive adhesive is still less liable to exude to the engaging surface, and flexibility is more satisfactory.

The density of the hook-and-loop fastener female member of the present invention is preferably 110 kg/m$^3$ or less, more preferably from 5 kg/m$^3$ to 110 kg/m$^3$, still more preferably from 10 kg/m$^3$ to 110 kg/m$^3$, still more preferably from 10 kg/m$^3$ to 80 kg/m$^3$, still more preferably from 10 kg/m$^3$ to 70 kg/m$^3$, particularly preferably from 10 kg/m$^3$ to 60 kg/m$^3$, most preferably from 20 kg/m$^3$ to 50 kg/m$^3$. When the density of the hook-and-loop fastener female member of the present invention falls within the above-mentioned range, the engaging force with a hook-and-loop fastener male member is more excellent, and hence, in a disposable diaper or the like, the problem of slippage, for example, at the time of wearing or after excretion canbe effectively eliminated. When the density of the hook-and-loop fastener female member of the present invention is more than 110 kg/m$^3$, a state in which the fiber of the non-woven fabric of the hook-and-loop fastener female member is densely packed is established, and hence there is a fear that it may be difficult to insert the engaging portion of a hook-and-loop fastener male member into the hook-and-loop fastener female member and an excellent engaging force cannot be expressed. The density (kg/m$^3$) of the hook-and-loop fastener female member of the present invention is a value calculated from the basis weight (X g/m$^2$) of the non-woven fabric in the hook-and-loop fastener female member of the present invention and the thickness (Y mm) of the non-woven fabric in the hook-and-loop fastener female member of the present invention measured on the basis of a method to be described later. More specifically, the density (kg/m$^3$) of the hook-and-loop fastener female member of the present invention is calculated as X/Y (kg/m$^3$) .

In the hook-and-loop fastener female member of the present invention, it is preferred that the surface of the fiber forming the non-woven fabric included in the engaging layer and the surface of the physical property layer on an engaging layer side contain the same kind of polymer. When the surface of the fiber forming the non-woven fabric included in the engaging layer and the surface of the physical property layer on the engaging layer side contain the same kind of polymer, there can be provided a hook-and-loop fastener female member more excellent in engaging force with a hook-and-loop fastener male member. In this case, the "surface of the fiber forming the non-woven fabric included in the engaging layer" may be any surface of the fiber, and encompasses, for example, a sheath portion in fiber having a core-sheath structure.

When the surface of the fiber forming the non-woven fabric included in the engaging layer and the surface of the physical property layer on the engaging layer side contain the same kind of polymer, any appropriate polymer may be adopted as the polymer as long as the effect of the present invention is not impaired. Such polymer is preferably polyolefin. When the surface of the fiber forming the non-woven fabric included in the engaging layer and the surface of the physical property layer on the engaging layer side contain the same kind of polyolefin, there can be provided a hook-and-loop fastener female member more excellent in engaging force with a hook-and-loop fastener male member.

The hook-and-loop fastener female member of the present invention has a flexibility index A, which is expressed by Equation (1), of 0.004 g$^{-2}$ or more.

$$A=1/(P1 \times P2) \quad (1)$$

In the equation, P1 represents a maximum compressive load when a ring body, which is obtained by cutting the hook-and-loop fastener female member into a size of 25 mm in an MD direction by 50 mm in a CD direction, forming the cut hook-and-loop fastener female member into a ring shape with its physical property layer surface being a roll inner side and its CD being a circumference, and connecting a front end portion and a rear end portion of the hook-and-loop fastener female member to each other at two places through use of a stapler in such a manner that the end portions do not overlap each other so as to form a ring shape having a roll diameter of 20 mm, is deformed through application of a compressive load to the ring body from its axial direction at a speed of 50 mm/minute up to 14 mm. In addition, P2 represents a maximum compressive load when a ring body, which is obtained by cutting the hook-and-loop fastener female member into a size of 25 mm in an MD direction by 50 mm in a CD direction, forming the cut hook-and-loop fastener female member into a ring shape with its physical property layer surface being a roll inner side and its CD being a circumference, and connecting the front end portion and the rear end portion of the hook-and-loop fastener female member to each at two places other through use of a stapler in such a manner that the end portions do not overlap each other so as to form a ring shape having a roll diameter of 20 mm, is deformed through application of a compressive load to the ring body from its width direction by allowing a plastic plate to fall freely at a height of 35 mm down to 5 mm. More specific measurement methods for P1 and P2 are described later.

The flexibility index A of the hook-and-loop fastener female member of the present invention, which is expressed by Equation (1), is 0.004 g$^{-2}$ or more, preferably from 0.004 g$^{-2}$ to 0.5 g$^{-2}$, more preferably from 0.006 g$^{-2}$ to 0.3 g$^{-2}$, still more preferably from 0.008 g$^{-2}$ to 0.1 g$^{-2}$, still more preferably from 0.01 g$^{-2}$ to 0.08 g$^{-2}$, particularly preferably from 0.015 g$^{-2}$ to 0.05 g$^{-2}$, most preferably from 0.02 g$^{-2}$ to 0.05 g$^{-2}$. When the flexibility index A falls within the above-mentioned range, there can be provided a hook-and-loop fastener female member excellent in engaging force with a hook-and-loop fastener male member. When the flexibility index A falls outside the above-mentioned range, there is a fear that the engaging force with a hook-and-loop fastener male member may lower.

The inventors of the present invention have surprisingly found that the engaging force with a hook-and-loop fastener male member is improved by adjusting the flexibility index A within the above-mentioned specific range, and thus have completed the present invention. It is surmised that, probably, when the flexibility index A is adjusted within the specific range, the hook-and-loop fastener female member becomes moderately sufficiently flexible, and as a result, its engaging area with a hook-and-loop fastener male member is sufficiently secured to improve the engaging force with the hook-and-loop fastener male member. In addition, it is surmised that, when the flexibility index A is so low as to fall outside the specific range, the flexibility of the hook-and-loop fastener female member becomes not sufficient, and hence the hook-and-loop fastener female member does not easily deform into a three-dimensional curved surface shape that may be formed in actual use, with the result that the hook-and-loop fastener male member cannot engage with the hook-and-loop fastener female member over a wide area. It is surmised that, when the flexibility index A is so large as to fall outside the specific range, the hook-and-loop fastener female member becomes excessively soft, with the result that handleability in actual use lowers, and moreover, the hook-and-loop fastener male member cannot engage with the hook-and-loop fastener female member successfully.

P1 is preferably 70 g or less, more preferably from 1 g to 60 g, still more preferably from 5 g to 50 g, particularly preferably from 10 g to 40 g, most preferably from 15 g to 40 g. When P1 falls within the above-mentioned range, there can be provided a hook-and-loop fastener female member more excellent in engaging force with a hook-and-loop fastener male member. When P1 falls outside the above-mentioned range, there is a fear that the engaging force with a hook-and-loop fastener male member may lower.

P2 is preferably 4.05 g or less, more preferably from 0.1 g to 4 g, still more preferably from 0.5 g to 3.5 g, particularly preferably from 0.8 g to 3 g, most preferably from 1 g to 2.5 g. When P2 falls within the above-mentioned range, there can be provided a hook-and-loop fastener female member more excellent in engaging force with a hook-and-loop fastener male member. When P2 falls outside the above-mentioned range, there is a fear that the engaging force with a hook-and-loop fastener male member may lower.

The hook-and-loop fastener female member of the present invention has an embossed pattern. Such embossed pattern is preferably formed by embossing treatment. Specific examples of the embossed pattern include a continuous grid shape, a discontinuous grid shape, a continuous curve shape, a discontinuous curve shape, a continuous zigzag shape, a discontinuous zigzag shape, a continuous linear shape, a discontinuous linear shape, a circle shape, an ellipse shape, a hollow circle shape, a hollow ellipse shape, an arc shape, and a hollow arc shape.

The embossed pattern of the hook-and-loop fastener female member of the present invention is preferably a discontinuous embossed pattern, more preferably an embossed pattern of an arc shape, because the effect of the present invention can be more effectively expressed. A schematic plan view of the hook-and-loop fastener female member of the present invention having the embossed pattern of an arc shape is illustrated in FIG. 1. In FIG. 1, a hook-and-loop fastener female member 100 of the present invention has a plurality of embossments **10** forming an embossed pattern of an arc shape. In FIG. 1, the hook-and-loop fastener female member of the present invention has a region **20** free of the embossed pattern. In the embossed pattern of an arc shape, it is preferred that individual embossments be embossments having no "corners".

In the hook-and-loop fastener female member of the present invention, when, for example, a spunbonded non-woven fabric or an air-through non-woven fabric is used as the non-woven fabric of fiber included in the engaging layer, pieces of the fiber forming the non-woven fabric included in the engaging layer can have mutual bonding points. With this, when the hook-and-loop fastener female member of the present invention has an embossed pattern, not only pieces of the fiber forming the non-woven fabric included in the engaging layer have firm mutual bonding points in the embossedpattern portions (the portions of the plurality of embossments 10 in FIG. 1) as a result of embossing treatment, but also pieces of the fiber forming the non-woven fabric included in the engaging layer have mutual bonding points in the region free of the embossed pattern (the region 20 free of the embossed pattern in FIG. 1). When such structure can be achieved, the hook-and-loop fastener female member of the present invention becomes more excellent in engaging force with a hook-and-loop fastener male member.

In the hook-and-loop fastener female member of the present invention, the embossment width of each of the plurality of embossments forming the embossed pattern is preferably from 0.1 mm to 3.0 mm, more preferably from 0.3 mm to 2.0 mm, still more preferably from 0.3 mm to 1.5 mm, particularly preferably from 0.5 mm to 1.5mm, most preferably from 0.5 mm to 1.0mm. When the embossment width falls within the above-mentioned range, the hook-and-loop fastener female member of the present invention becomes more excellent in engaging force with a hook-and-loop fastener male member. The embossment width in the hook-and-loop fastener female member of the present invention refers to, for example, a width W of each of the embossments 10 in an MD direction as illustrated in FIG. 1.

In the hook-and-loop fastener female member of the present invention, the distance between two adjacent embossments in the plurality of embossments forming the embossed pattern on any line in the MD direction is preferably 10 mm or less, more preferably from 1 mm to 10 mm, still more preferably from 1.5 mm to 9 mm, particularly preferably from 2 mm to 8 mm, most preferably from 2.5 mm to 7 mm. When the distance between two adjacent embossments in the plurality of embossments forming the embossed pattern on any line in the MD direction falls within the above-mentioned range, the hook-and-loop fastener female member of the present invention becomes more excellent in engaging force with a hook-and-loop fastener male member. The distance between two adj acent embossments in the plurality of embossments forming the embossed pattern on a line in the MD direction in the hook-and-loop fastener female member of the present invention is, for example, a distance L between two adjacent embossments on a line P in the MD direction illustrated in FIG. 1 (which may be a line in the MD direction at any position in a CD direction). That is, the distance L is the maximum value of the distance between two adj acent embossments (sometimes referred to as "maximum embossment-to-embossment distance") on a line in the MD direction in the embossed pattern (which may be a line in the MD direction at any position in the CD direction).

The depth of each of the embossments is preferably from 0.1 mm to 2.0 mm, more preferably from 0.2 mm to 1.8 mm, still more preferably from 0.3 mm to 1.5 mm, particularly preferably from 0.5 mm to 1.5 mm, most preferably from 0.7 mm to 1.2 mm. When the depth of each of the embossments falls within the above-mentioned range, the hook-and-loop fastener female member of the present invention becomes more excellent in engaging force with a hook-and-loop fastener male member.

In the hook-and-loop fastener female member of the present invention, the ratio of the area of a welded portion formed by the embossed pattern to the area of the entire surface of the hook-and- loop fastener female member (hereinafter sometimes referred to as "embossing-welded area ratio") is preferably 35% or less, more preferably from 5% to 33%, still more preferably from 10% to 30%, particularly preferably from 12% to 28%, most preferably from 12% to 23%. When the embossing-welded area ratio falls within the above-mentioned range, the hook-and-loop fastener female member of the present invention becomes more excellent in engaging force with a hook-and-loop fastener male member.

In the hook-and-loop fastener female member of the present invention, the thickness of the welded portion formed by the embossed pattern is 100 $\mu$m or less, more preferably from 10 $\mu$m to 100 $\mu$m, still more preferably from 15 $\mu$m to 80 $\mu$m, still more preferably from 20 $\mu$m to 70 $\mu$m, particularly preferably from 25 $\mu$m to 60 $\mu$m, most preferably from 25 $\mu$m to 55 $\mu$m. When the thickness of the welded portion formed by the embossed pattern falls within the above-mentioned range, the hook-and-loop fastener female member of the present invention becomes more excellent in engaging force with a hook-and-loop fastener male member.

<<Production Method for Hook-and-loop Fastener Female Member of the Present Invention>>

As one preferred production method for the hook-and-loop fastener female member of the present invention, raw non-woven fabrics are laminated and subjected to embossing treatment with a pattern roll at any appropriate treatment temperature (e.g., 160°C), any appropriate linear pressure (e.g., 160N/mm), and any appropriate treatment speed (e.g., 10 m/minute) to provide a desired hook-and-loop fastener female member.

As another preferred production method for the hook-and-loop fastener female member of the present invention, raw non-woven fabrics are laminated and subjected to embossing treatment with an ultrasonic welding machine at any appropriate frequency (e.g. , 20 kHz (output intensity: 1,800 W)) and any appropriate treatment speed (e.g., 50m/minute) to provide a desired hook-and-loop fastener female member.

<<Application of Hook-and-loop Fastener Female Member of the Present Invention>>

The hook-and-loop fastener female member of the present invention can provide a hook-and-loop fastener by being combined with a hook-and-loop fastener male member configured to engage with the hook-and-loop fastener female member. That is, a hook-and-loop fastener of the present invention includes the hook-and-loop fastener female member of the present invention and a hook-and-loop fastener male member configured to engage with the hook-and-loop fastener female member. In addition, the hook-and-loop fastener female member of the present invention can be used in any appropriate article in which the effect of the present invention can be effectively utilized. A typical example of such article is a sanitary article. That is, a sanitary article of the present invention includes the hook-and-loop fastener female member of the present invention. Examples of such sanitary article include a diaper (in particular, a disposable diaper), a supporter, and a mask.

Examples

The present invention is hereinafter specifically described by way of Examples. However, the present invention is by no means limited to these Examples. In Examples and the like, test and evaluation methods are as described below. In addition, "part (s) " means "part (s) by weight" and "%" means "wt%" unless otherwise stated.

<Flexibility Index A>

(Production of Ring Body)

An obtained hook-and-loop fastener female member was cut into a size of 25 mm in an MD direction by 50 mm in a CD direction. The cut hook-and-loop fastener female member was formed into a ring shape with its physical property layer surface being a roll inner side and its CD being a circumference, and end portions thereof were fixed to each other at two places through use of a stapler in such a manner that the end portions did not overlap each other.

(Measurement of P1)

A compressive load was applied to the resultant ring body of the hook-and-loop fastener female member from its axial direction at a speed of 50 mm/minute up to 14 mm, and the maximum compressive load when the ring body was deformed was measured. Technograph TG-2kN manufactured by Minebea Co., Ltd. was used as a measuring machine. This operation was repeated a total of 3 times, and the average value of the 3 measured values was defined as P1.

(Measurement of P2)

A compressive load was applied to the resultant ring body of the hook-and-loop fastener female member from its width direction by allowing a plastic plate to fall freely at a height of 35 mm down to 5 mm, and the maximum compressive load when the ring body was deformed was measured. GX-300 manufactured by A & D Company, Limited was used as a measuring machine. This operation was repeated a total of 3 times, and the average value of the 3 measured values was defined as P2.

(Calculation of Flexibility Index A)

A flexibility index A was calculated in accordance with $A = 1/(P1 \times P2)$.

<Evaluation of Engaging Property>

An obtained hook-and-loop fastener female member was cut into a size of 5 cm$\times$5 cm and allowed to stand still on a commercially available disposable diaper. Meanwhile, a 25 mm$\times$13 mm male member having a guide made of a polyethylene film having a thickness of 60 $\mu$m (commercially available mechanical hook member for a hook-and-loop fastener) was prepared, and was allowed to stand still on the female member so that its 25 mm sides were parallel to the flow direction of the engaging layer of the female member, followed by pressure bonding with a finger. After that, the guide was manually peeled and an engaging property was checked.
Evaluation was performed in accordance with the following criteria.

○○○: The engaging property is extremely strong.
○○: The engaging property is sufficiently strong.
○: The engaging property is sufficient.
×: The engaging property is insufficient, or engagement does not occur.

<Measurement of Fiber Diameter>

Through the use of a digital microscope "VHX-1000" manufactured by Keyence Corporation, a non-woven fabric surface was photographed at a magnification of 500, and fiber diameters were measured for N=5 or more with the image analysis software of the digital microscope. The average value of the measured fiber diameters was defined as a fiber diameter.

<Thickness of Non-woven Fabric>

Through the use of a digital microscope "VHX-1000" manufactured by Keyence Corporation, a cross-section of a non-woven fabric was photographed at a magnification of 100, and the thickness of the non-woven fabric was measured with the image analysis software of the digital microscope. In the measurement of the thickness, parallel lines drawn so as to have 10 points of intersection with fiber in an upper portion and a lower portion of the cross-section of the non-woven fabric, respectively, were defined as an upper side and a lower side, respectively, and a length between the upper side and the lower side was defined as the thickness of the non-woven fabric.

[Examples 1 to 11 and Comparative Examples 1 to 3]

Raw non-woven fabrics shown in Table 1 and Table 2 were laminated, and were subjected to embossing treatment with an embossed pattern roll at a treatment temperature of 200°C, a linear pressure of 160 N/mm, and a treatment speed of 20 m/minute to provide hook-and-loop fastener female members.
The embossed pattern used was the embossed pattern of an arc shape illustrated in FIG. 1, and the embossment width, the maximum value of the distance between two adjacent embossments on a line in the MD direction (maximum embossment-to-embossment distance), the embossing-welded area ratio, and the thickness of a weldedportion formed by the embossed pattern were as shown in Table 1 and Table 2.

[Examples 12 and 13]

Raw non-woven fabrics shown in Table 2 were laminated and subjected to embossing treatment with an ultrasonic welding machine (manufactured by Herrmann Ultrasonics, apparatus name: MICROBOND (ULTRABOND 48:20)) at a frequency of 20 kHz (output intensity: 1,800 W) and a treatment speed of 50 m/minute to provide hook-and-loop fastener female members.
The embossed pattern used was the embossed pattern of an arc shape illustrated in FIG. 1, and the embossment width, the maximum value of the distance between two adjacent embossments on a line in the MD direction (maximum embossment-to-embossment distance), the embossing-welded area ratio, and the thickness of a weldedportion formed by the embossed pattern were as shown in Table 2.

Table 1

| | | Example 1 | Example 2 | Example 3 | Example 4 | Example 5 | Example 6 | Example 7 | Example 8 |
|---|---|---|---|---|---|---|---|---|---|
| Engaging layer | Construction | Non-woven fabric | Non-woven fabric | Non-woven fabric | Non-woven fabric | Non-woven fabric | Non-woven fabric | Non-woven fabric | Non-woven fabric |
| | Fiber composition | PP | PP | PP | PE/PP | PE/PP | PE/PET | PE/PET | PE/PET |
| | Fiber diameter [μm] | 21.8 | 21.8 | 21.8 | 25.2 | 20.0 | 31.6 | 27.2 | 31.3 |
| | Basis weight [g/m²] | 18 | 18 | 18 | 30 | 18 | 30 | 40 | 20 |
| | Thickness of region free of embossed pattern [mm] | 0.19 | 0.19 | 0.19 | 0.96 | 0.83 | 1.12 | 1.17 | 2.19 |
| | Density [kg/m³] | 94 | 94 | 94 | 31 | 22 | 27 | 34 | 9 |
| Physical property layer | Construction | Non-woven fabric | Non-woven fabric | Non-woven fabric | Non-woven fabric | Non-woven fabric | Non-woven fabric | Non-woven fabric | Non-woven fabric |
| | Fiber composition | PP | PP | PP | PE/PP | PE/PP | PE/PP | PE/PP | PE/PP |
| | Fiber diameter [μm] | 19.6 | 19.6 | 9.0 | 19.8 | 19.8 | 19.8 | 19.8 | 19.8 |
| | Basis weight [g/m²] | 14 | 20 | 15 | 15 | 15 | 15 | 15 | 15 |
| | Thickness of region free of embossed pattern [mm] | 0.15 | 0.18 | 0.12 | 0.14 | 0.14 | 0.14 | 0.14 | 0.14 |
| | Density [kg/m³] | 93 | 111 | 125 | 107 | 107 | 107 | 107 | 107 |
| Lamination of engaging layer and physical property layer | | Embossing welding | Embossing welding | Embossing welding | Embossing welding | Embossing welding | Embossing welding | 107 Embossing welding | 107 Embossing welding |
| Total basis weight [g/m²] | | 32 | 38 | 33 | 45 | 33 | 45 | 55 | 35 |
| Total density [kg/m³] | | 94 | 102 | 106 | 41 | 34 | 36 | 42 | 15 |
| Shape of embossed pattern | | Discontinuous | Discontinuous | Discontinuous | Discontinuous | Discontinuous | Discontinuous | Discontinuous | Discontinuous |
| Embossment width [mm] | | 0.8 | 0.8 | 0.8 | 0.8 | 0.8 | 0.8 | 0.8 | 0.8 |
| Maximum embossment-to-embossment distance [mm] | | 3.2 | 3.2 | 3.2 | 3.2 | 3.2 | 3.2 | 3.2 | 3.2 |

(continued)

| | Example 1 | Example 2 | Example 3 | Example 4 | Example 5 | Example 6 | Example 7 | Example 8 |
|---|---|---|---|---|---|---|---|---|
| Embossing-welded area ratio [%] | 23 | 23 | 23 | 23 | 23 | 23 | 23 | 23 |
| Thickness of welded portion formed by embossed pattern [μm] | 25 | 33 | 30 | 40 | 30 | 43 | 52 | 40 |
| P1 [g] | 18.69 | 39.08 | 21.75 | 27.86 | 17.67 | 23.45 | 36.36 | 17.67 |
| P2 [g] | 1.53 | 2.43 | 1.50 | 2.40 | 1.37 | 2.07 | 4.03 | 1.53 |
| Flexibility index A [g$^{-2}$] | 0.035 | 0.011 | 0.031 | 0.015 | 0.041 | 0.021 | 0.007 | 0.037 |
| Engaging property | ○○ | ○ | ○○ | ○○ | ○○○ | ○○ | ○ | ○○○ |

Table 2

| | | Example 9 | Example 10 | Example 11 | Example 12 | Example 13 | Comparative Example 1 | Comparative Example 2 | Comparative Example 3 |
|---|---|---|---|---|---|---|---|---|---|
| Engaging layer | Construction | Non-woven fabric | Non-woven fabric | Non-woven fabric | Non-woven fabric | Non-woven fabric | Non-woven fabric | Non-woven fabric | Non-woven fabric |
| | Fiber composition | PP | PP | PP | PP | PP | PP | PP | PP |
| | Fiber diameter [μm] | 14.5 | 14.5 | 14.5 | 21.8 | 14.5 | 19.6 | 22.5 | 23.2 |
| | Basis weight [g/m²] | 24 | 24 | 24 | 18 | 24 | 20 | 70 | 65 |
| | Thickness of region free of embossed pattern [mm] | 0.42 | 0.42 | 0.42 | 0.19 | 0.42 | 0.18 | 0.80 | 0.75 |
| | Density [kg/m³] | 57 | 57 | 57 | 94 | 57 | 111 | 88 | 87 |
| Physical property layer | Construction | Non-woven fabric | Non-woven fabric | Non-woven fabric | Non-woven fabric | Non-woven fabric | Film | - | - |
| | Fiber composition | PP | PP | PP | PP | PP | PP | - | - |
| | Fiber diameter [μm] | 19.6 | 19.6 | 9.0 | 19.6 | 19.6 | - | - | - |
| | Basis weight [g/m²] | 14 | 20 | 15 | 14 | 14 | 25 | - | - |
| | Thickness of region free of embossed pattern [mm] | 0.15 | 0.18 | 0.12 | 0.15 | 0.15 | - | - | - |
| | Density [kg/m³] | 93 | 111 | 125 | 93 | 93 | - | - | - |
| Lamination of engaging layer and physical property layer | | Embossing welding | Embossing welding | Embossing welding | Embossing welding | Embossing welding | Embossing welding | Embossing welding | Embossing welding |
| Total basis weight [g/m²] | | 38 | 44 | 39 | 32 | 38 | 45 | 70 | 65 |
| Total density [kg/m³] | | 67 | 73 | 72 | 94 | 67 | 220 | 88 | 87 |
| Shape of embossed pattern | | Discontinuous | Discontinuous | Discontinuous | Discontinuous | Discontinuous | Discontinuous | Continuous | Discontinuous |
| Embossment width [mm] | | 0.8 | 0.8 | 0.8 | 0.6 | 0.6 | 0.8 | 0.8 to 4.0 | 2.5 |
| Maximum embossment-to-embossment distance [mm] | | 3.2 | 3.2 | 3.2 | 3.4 | 3.4 | 3.2 | 4.0 | 3.5 |

(continued)

| | Example 9 | Example 10 | Example 11 | Example 12 | Example 13 | Comparative Example 1 | Comparative Example 2 | Comparative Example 3 |
|---|---|---|---|---|---|---|---|---|
| Embossing-welded area ratio [%] | 23 | 23 | 23 | 17 | 17 | 23 | 30 | 39 |
| Thickness of welded portion formed by embossed pattern [μm] | 36 | 45 | 39 | 20 | 32 | - | 60 | 95 |
| P1 [g] | 18.52 | 30.02 | 26.22 | 16.25 | 18.37 | 73.73 | 166.16 | 91.22 |
| P2 [g] | 1.82 | 2.00 | 2.01 | 1.21 | 1.53 | 4.17 | 9.10 | 4.51 |
| Flexibility index A [g$^{-2}$] | 0.030 | 0.017 | 0.019 | 0.051 | 0.036 | 0.003 | 0.001 | 0.002 |
| Engaging property | ○○ | ○○ | ○○ | ○ | ○○○ | × | × | × |

The hook-and-loop fastener female member of the present invention can be used for any appropriate article in which the effect of the present invention can be effectively utilized. A typical example of such article is a sanitary article. Examples of such sanitary article include a diaper (in particular, a disposable diaper), a supporter, and a mask.

Reference Signs List

**100** hook-and-loop fastener female member
**10** embossment
**20** region free of embossed pattern

**Claims**

1. A hook-and-loop fastener female member, comprising:

   an engaging layer engageable with a hook-and-loop fastener male member; and
   a physical property layer configured to hold the engaging layer,
   wherein the engaging layer includes a non-woven fabric of fiber,
   wherein a material for the physical property layer is a non-woven fabric of fiber,
   wherein the hook-and-loop fastener female member has an embossed pattern,
   wherein the thickness of the welded portion formed by the embossed pattern is 100 $\mu$m or less, and
   wherein the hook-and-loop fastener female member has a flexibility index A, which is expressed by Equation (1), of 0.004 g$^{-2}$ or more:

$$A=1/(P1 \times P2) \quad (1)$$

   where:

   P1 represents a maximum compressive load when a ring body, which is obtained by cutting the hook-and-loop fastener female member into a size of 25 mm in an MD direction by 50 mm in a CD direction, forming the cut hook-and-loop fastener female member into a ring shape with its physical property layer surface being a roll inner side and its CD being a circumference, and connecting a front end portion and a rear end portion of the hook-and-loop fastener female member to each other at two places through use of a stapler in such a manner that the end portions do not overlap each other so as to have a ring shape having a roll diameter of 20 mm, is deformed through application of a compressive load to the ring body from its axial direction at a speed of 50 mm/minute up to 14 mm; and
   P2 represents a maximum compressive load when the ring body, which is obtained by cutting the hook-and-loop fastener female member into a size of 25 mm in an MD direction by 50 mm in a CD direction, forming the cut hook-and-loop fastener female member into a ring shape with its physical property layer surface being a roll inner side and its CD being a circumference, and connecting the front end portion and the rear end portion of the hook-and-loop fastener female member to each other at two places through use of a stapler in such a manner that the end portions do not overlap each other so as to have a ring shape having a roll diameter of 20 mm, is deformed through application of a compressive load to the ring body from its width direction by allowing a plastic plate to fall freely at a height of 35 mm down to 5 mm.

2. The hook-and-loop fastener female member according to claim 1, wherein the flexibility index A is from 0.01 g$^{-2}$ to 0.08 g$^{-2}$, preferably from 0.02 g$^{-2}$ to 0.05 g$^{-2}$.

3. The hook-and-loop fastener female member according to claim 1, wherein the P1 is 70 g or less, preferably from 15 g to 40 g.

4. The hook-and-loop fastener female member according to claim 1, wherein the P2 is 4.05 g or less, preferably from 1 g to 2.5 g.

5. The hook-and-loop fastener female member according to claim 1, wherein a sum of a basis weight of the non-woven fabric in the engaging layer and a basis weight of a non-woven fabric in the physical property layer is 60 g/m$^2$ or less, preferably from 30 g/m$^2$ to 47 g/m$^2$.

6. The hook-and-loop fastener female member according to claim 1, wherein the hook-and-loop fastener female member has a density of 110 kg/m$^3$ or less, preferablyof 50 kg/m$^3$ or less.

7. The hook-and-loop fastener female member according to claim 1, wherein the hook-and-loop fastener female member is formed only of non-woven fabrics.

8. The hook-and-loop fastener female member according to claim 1, wherein a ratio of an area of a welded portion formed by the embossed pattern to an area of an entire surface of the hook-and-loop fastener female member is 35% or less, preferably from 12% to 28%.

9. The hook-and-loop fastener female member according to claim 1, wherein a welded portion formed by the embossed pattern has a thickness of from 25 $\mu$m to 55 $\mu$m.

10. The hook-and-loop fastener female member according to claim 1, wherein the embossed pattern comprises a discontinuous embossed pattern.

11. The hook-and-loop fastener female member according to claim 1, wherein a surface of the fiber forming the non-woven fabric included in the engaging layer and a surface of the physical property layer on an engaging layer side contain the same kind of polymer.

12. The hook-and-loop fastener female member according to claim 11, wherein the polymer comprises polyolefin.

13. A hook-and-loop fastener, comprising:

the hook-and-loop fastener female member of claim 1; and
a hook-and-loop fastener male member configured to engage with the hook-and-loop fastener female member.

14. A sanitary article, comprising the hook-and-loop fastener female member of claim 1.

**Patentansprüche**

1. Aufnahmeelement eines Klettverschlusses, umfassend:

eine Verbindungsschicht, die mit einem Einsteckelement des Klettverschlusses in Verbindung gebracht werden kann; und
eine physikalische Eigenschaftsschicht, die zum Halten der Verbindungsschicht ausgebildet ist,
wobei die Verbindungsschicht einen Faservliesstoff enthält,
wobei ein Material für die physikalische Eigenschaftsschicht ein Faservliesstoff ist,
wobei das Aufnahmeelement des Klettverschlusses ein Prägemuster aufweist,
wobei die Dicke des geschweißten Abschnitts, der durch das Prägemuster gebildet wird, 100 $\mu$m oder weniger beträgt, und
wobei das Aufnahmeelement des Klettverschlusses einen Flexibilitätsindex A, der durch Gleichung (1) ausgedrückt wird, von 0,004 g$^{-2}$ oder mehr aufweist:

$$A=1/(P1\times P2) \quad (1)$$

wobei:

P1 eine maximale Druckbelastung darstellt, wenn ein Ringkörper, der durch das Schneiden des Aufnahmeelements des Klettverschlusses in eine Größe von 25 mm in eine MD-Richtung mal 50 mm in eine CD-Richtung, das Formendes geschnittenen Auf nahmeelements des Klettverschlusses in eine Ringform, wobei seine Oberfläche der physikalischen Eigenschaftsschicht eine Rolleninnenseite ist und sein CD ein Umfang ist, und das Verbinden eines vorderen Endabschnitts und eines hinteren Endabschnitts des Aufnahmeelements des Klettverschlusses miteinander an zwei Stellen durch Verwendung eines Hefters in einer solchen Weise, dass die Endabschnitte einander nicht überlappen, um eine Ringform mit einem Rollendurchmesser von 20 mm aufzuweisen, gebildet wird, durch Aufbringen einer Druckbelastung auf den Ringkörper aus seiner axialen Richtung mit einer Geschwindigkeit von 50 mm/Minute bis zu 14 mm verformt wird; und

P2 eine maximale Druckbelastung darstellt, wenn der Ringkörper, der durch das Schneiden des Aufnahmeelementes des Klettverschlusses in eine Größe von 25 mm in eine MD-Richtungmal 50 mm in eine CD-Richtung, das Formen des geschnittenen Aufnahmeelementes des Klettverschlusses in eine Ringform, wobei seine Oberfläche der physikalischen Eigenschaftsschicht eine Rolleninnenseite ist und sein CD ein Umfang ist, und das Verbinden des vorderen Endabschnitts und des hinteren Endabschnitts des Aufnahmeelements des Klettverschlusses miteinander an zwei Stellen durch Verwendung eines Hefters in einer solchen Weise, dass die Endabschnitte einander nicht überlappen, um eine Ringform mit einem Rollendurchmesser von 20 mm aufzuweisen, gebildet wird, durch Aufbringen einer Druckbelastung auf den Ringkörper aus seiner Breitenrichtung durch Freifallenlassen einer Kunststoffplatte aus einer Höhe von 35 mm bis zu 5 mm verformt wird.

2. Aufnahmeelement eines Klettverschluss nach Anspruch 1, wobei der Flexibilitätsindex A von 0,01 $g^{-2}$ bis 0,08 $g^{-2}$ beträgt, vorzugsweise von 0,02 $g^{-2}$ bis 0,05 $g^{-2}$.

3. Aufnahmeelement eines Klettverschlusses nach Anspruch 1, wobei der P1 70 g oder weniger beträgt, vorzugsweise von 15 g bis 40 g.

4. Aufnahmeelement eines Klettverschlusses nach Anspruch 1, wobei der P2 4,05 g oder weniger beträgt, vorzugsweise von 1 g bis 2,5 g.

5. Aufnahmeelement eines Klettverschlusses nach Anspruch 1, wobei eine Summe eines Basisgewichtes des Vliesstoffes in der Verbindungsschicht und eines Basisgewichtes eines Vliesstoffes in der physikalischen Eigenschaftsschicht 60 $g/m^2$ oder weniger beträgt, vorzugsweise von 30 $g/m^2$ bis 47 $g/m^2$.

6. Aufnahmeelement eines Klettverschlusses nach Anspruch 1, wobei das Aufnahmeelement des Klettverschlusses eine Dichte von 110 $kg/m^3$ oder weniger aufweist, vorzugsweise von 50 $kg/m^3$ oder weniger.

7. Aufnahmeelement eines Klettverschlusses nach Anspruch 1, wobei das Aufnahmeelement des Klettverschlusses nur aus Vliesstoffen gebildet ist.

8. Aufnahmeelement eines Klettverschlusses nach Anspruch 1, wobei ein Verhältnis einer Fläche eines geschweißten Abschnitts, der durch das Prägemuster gebildet wird, zu einer Fläche einer gesamten Oberfläche des Aufnahmeelementes des Klettverschlusses 35 % oder weniger beträgt, vorzugsweise von 12 % bis 28 %.

9. Aufnahmeelement eines Klettverschlusses nach Anspruch 1, wobei ein geschweißter Abschnitt, der durch das Prägemuster gebildet wird, eine Dicke von 25 $\mu$m bis 55 $\mu$m aufweist.

10. Aufnahmeelement eines Klettverschlusses nach Anspruch 1, wobei das Prägemuster ein diskontinuierliches Prägemuster umfasst.

11. Aufnahmeelement eines Klettverschlusses nach Anspruch 1, wobei eine Oberfläche der Faser, die den Vliesstoff bildet, der in der Verbindungsschicht enthalten ist, und eine Oberfläche der physikalischen Eigenschaftsschicht auf einer Verbindungsschichtseite die gleiche Art von Polymer enthalten.

12. Aufnahmeelement eines Klettverschlusses nach Anspruch 11, wobei das Polymer Polyolefin umfasst.

13. Klettverschluss, umfassend:

das Aufnahmeelement eines Klettverschlusses nach Anspruch 1; und
ein Einsteckelement des Klettverschlusses, ausgebildet um sich mit dem Aufnahmeelement des Klettverschlusses zu verbinden.

14. Hygieneartikel, umfassend das Aufnahmeelement eines Klettverschlusses nach Anspruch 1.

**Revendications**

1. Élément femelle d'une fixation par crochets et boucles, comprenant:

   une couche prenante pouvant être en prise avec un élément mâle d'une fixation par crochets et boucles; et
   une couche de propriété physique réalisée pour maintenir la couche prenante, dans lequel la couche prenante comprend un tissu non tissé de fibre,
   dans lequel une matière pour la couche de propriété physique est un tissu non tissé de fibre,
   dans lequel l'élément femelle d'une fixation par crochets et boucles présente un motif en relief,
   dans lequel l'épaisseur de la partie soudée formée par le motif en relief fait 100 μm ou moins, et
   dans lequel l'élément femelle d'une fixation par crochets et boucles présente un indice de flexibilité A, lequel est exprimé par l'équation (1), de 0,004 g$^{-2}$ ou plus :

$$A=1/(P1 \times P2) \quad (1)$$

   où:

   P1 représente une charge de compression maximale quand un corps annulaire, lequel est obtenu par découpe de l'élément femelle d'une fixation par crochets et boucles en une dimension de 25 mm dans le sens MD sur 50 mm dans le sens CD, formation de l'élément femelle d'une fixation par crochets et boucles découpé en une forme annulaire avec sa couche de propriété physique qui est un côté intérieur de bobine et son CD étant une circonférence, et raccord d'une partie terminale avant et d'une partie terminale arrière de l'élément femelle d'une fixation par crochets et boucles l'un à l'autre en deux points par l'utilisation d'une agrafeuse de telle sorte que les parties terminales ne se recouvrent pas l'une l'autre de manière à avoir une forme annulaire d'un diamètre de bobine de 20 mm, est déformé par l'application d'une charge compressive au corps annulaire de sa direction axiale à une vitesse de 50 mm/minute jusqu'à 14 mm ; et
   P2 représente une charge de compression maximale quand le corps annulaire, lequel est obtenu par découpe de l'élément femelle d'une fixation par crochets et boucles en une dimension de 25 mm dans le sens MD sur 50 mm dans le sens CD, formation de l'élément femelle d'une fixation par crochets et boucles découpé en une forme annulaire avec sa couche de propriété physique qui est un côté intérieur de bobine et son CD étant une circonférence, et raccord de la partie terminale avant et de la partie terminale arrière de l'élément femelle d'une fixation par crochets et boucles l'un à l'autre en deux points par l'utilisation d'une agrafeuse de telle sorte que les parties terminales ne se recouvrent pas l'une l'autre de manière à avoir une forme annulaire d'un diamètre de bobine de 20 mm, est déformé par l'application d'une charge compressive au corps annulaire de son sens de la largeur en permettant à une plaque de plastique de tomber librement à une hauteur de 35 mm jusqu'à 5 mm.

2. Élément femelle d'une fixation par crochets et boucles selon la revendication 1, dans lequel l'indice de flexibilité A va de 0,01 g$^{-2}$ à 0,08 g$^{-2}$, de préférence de 0,02 g$^{-2}$ à 0,05 g$^{-2}$.

3. Élément femelle d'une fixation par crochets et boucles selon la revendication 1, dans lequel le P1 fait 70 g ou moins, et va de préférence de 15 g à 40 g.

4. Élément femelle d'une fixation par crochets et boucles selon la revendication 1, dans lequel le P2 fait 4,05 g ou moins, et va de préférence de 1 g à 2,5 g.

5. Élément femelle d'une fixation par crochets et boucles selon la revendication 1, dans lequel la somme d'un poids de base du tissu non tissé dans la couche prenante et le poids de base d'un tissu non tissé dans la couche de propriété physique, est de 60 g/m$^2$ ou moins, et va de préférence de 30 g/m$^2$ à 47 g/m$^2$.

6. Élément femelle d'une fixation par crochets et boucles selon la revendication 1, dans lequel l'élément femelle d'une fixation par crochets et boucles présente une densité de 110 kg/m$^3$ ou moins, de préférence de 50 kg/m$^3$ ou moins.

7. Élément femelle d'une fixation par crochets et boucles selon la revendication 1, dans lequel l'élément femelle d'une fixation par crochets et boucles est formé seulement de tissus non tissés.

8. Élément femelle d'une fixation par crochets et boucles selon la revendication 1, dans lequel un rapport d'une zone de la partie soudée formée par le motif en relief, par rapport à une zone de toute la surface de l'élément femelle d'une fixation par crochets et boucles est de 35% ou moins, et va de préférence de 12% à 28%.

9. Élément femelle d'une fixation par crochets et boucles selon la revendication 1, dans lequel une partie soudée formée par le motif en relief présente une épaisseur et va de 25 $\mu$m à 55 $\mu$m.

10. Élément femelle d'une fixation par crochets et boucles selon la revendication 1, dans lequel le motif en relief comprend un motif en relief discontinu.

11. Élément femelle d'une fixation par crochets et boucles selon la revendication 1, dans lequel une surface de la fibre formant le tissu non tissé comprise dans la couche prenante et la surface de la couche de propriété physique sur un côté couche prenante contiennent le même type de polymère.

12. Élément femelle d'une fixation par crochets et boucles selon la revendication 11, dans lequel le polymère comprend une polyoléfine.

13. Fixation par crochets et boucles, comprenant:

l'élément femelle d'une fixation par crochets et boucles selon la revendication 1; et
un élément mâle d'une fixation par crochets et boucles configuré pour être en prise avec l'élément femelle d'une fixation par crochets et boucles.

14. Article sanitaire, comprenant l'élément femelle d'une fixation par crochets et boucles selon la revendication 1.

EP 3 311 688 B1

FIG. 1

22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008130807 A1
- US 200080026178 A1
- WO 9719808 A1
- WO 2007097467 A1
- JP 2009527315 A
- JP 2010125337 A

### Non-patent literature cited in the description

- **E.A. VAUGHN.** Nonwoven Fabric Primer and Reference Sampler. Association of the Nonwoven Fabrics Industry, 1992